Europäisches Patentamt

European Patent Office

Office européen des brevets

(19)

(11) Numéro de publication : **0 095 968**
**B1**

(12) **FASCICULE DE BREVET EUROPÉEN**

(45) Date de publication du fascicule du brevet :
12.11.86

(51) Int. Cl.⁴ : **A 61 K 9/50**, A 61 K 9/52,
B 01 J 13/02

(21) Numéro de dépôt : 83401009.2

(22) Date de dépôt : 20.05.83

(54) Microcapsules à parois mixtes formées de polyholosides et de protéines réticules ainsi que leur procédé de préparation.

(30) Priorité : 26.05.82 FR 8209156

(43) Date de publication de la demande :
07.12.83 Bulletin 83/49

(45) Mention de la délivrance du brevet :
12.11.86 Bulletin 86/46

(84) Etats contractants désignés :
AT BE CH DE FR GB IT LI LU NL SE

(56) Documents cités :
EP-A- 0 025 379
FR-A- 1 415 039
FR-A- 2 275 250
FR-A- 2 305 229
GB-A- 2 026 976
GB-A- 2 040 863
Le dossier contient des informations techniques présentées postérieurement au dépôt de la demande et ne figurant pas dans le présent fascicule.

(73) Titulaire : **CENTRE NATIONAL DE LA RECHERCHE SCIENTIFIQUE (CNRS)**
15, Quai Anatole France
F-75007 Paris (FR)

(72) Inventeur : **Levy, Marie-Christine**
18 ter rue Houzeau-Muivon
F-51100 Reims (FR)
Inventeur : **Gourdier, Bertrand**
Le Noyer des Enfants Les Mesneux
F-51500 Rilly La Montagne (FR)

(74) Mandataire : **Ahner, Francis et al**
CABINET REGIMBEAU 26, avenue Kléber
F-75116 Paris (FR)

Jouve, 18, rue St-Denis, 75001 Paris, France

# 0 095 968

**Description**

La présente invention concerne des microcapsules à parois mixtes obtenues par réticulation d'un mélange d'au moins un polyholoside hydrosoluble ou dérivé hydrosoluble de polyholoside et d'au moins une protéine, ainsi que leur procédé de préparation. L'invention se rapporte à des microcapsules renfermant en particulier, mais non exclusivement, une substance pharmaceutiquement active. Les microcapsules selon l'invention peuvent en effet également renfermer d'autres substances telles que des substances alimentaires et en particulier des huiles essentielles.

On rappellera tout d'abord brièvement que les microcapsules sont des organites artificiels qui présentent un très grand intérêt pour la mise en forme galénique de divers médicaments. L'inclusion d'un principe actif dans une sphérule microscopique permet en effet d'assurer sa protection transitoire vis-à-vis d'agents dénaturants, tels que les enzymes digestives.

Dans d'autres cas, la paroi des microcapsules module la diffusion du principe actif, ce qui est mis à profit dans l'élaboration de formes galéniques à action prolongée ou retardée. Il est nécessaire que la paroi de la sphérule présente toutes les garanties d'innocuité liées à l'emploi en thérapeutique humaine.

L'état de la technique concernant les microcapsules, et en particulier les microcapsules obtenues par un procédé de réticulation interfaciale peut être illustré par FR-A-2 305 229, FR-A-2 275 250, FR-A-1 415 039 et GB-A-2 040 863. En revanche, aucun de ces documents de technique antérieure ne suggère la fabrication de microcapsules à parois externes mixtes constituées à la fois de polyholosides réticulés et de protéine réticulée. L'état de la technique peut en outre être complété par EP-A-25 379 visant des microcapsules essentiellement destinées à des applications dans le domaine de l'agriculture. Ces microcapsules sont obtenues par conservation suivie d'une réticulation.

En revanche, l'objet de la présente invention permet de répondre parfaitement aux exigences thérapeutiques énoncées précédemment, et permet en particulier l'incorporation de principes actifs, hydrosolubles ou lyposolubles, dans des microcapsules gastrorésistantes et entérosolubles.

Conformément à la présente invention, les microcapsules renfermant une substance pharmaceutiquement active, gastrorésistantes et entérosolubles, du type présentant une paroi externe obtenue par réticulation interfaciale d'un polyholoside à l'aide d'un agent de réticulation constitué par un réactif bifonctionnel acylant, sont caractérisées en ce que la paroi externe mixte des microcapsules est obtenue par réticulation interfaciale d'un mélange comprenant d'une part au moins un polyholoside hydrosoluble ou dérivé hydrosoluble de polyholoside et d'autre part au moins une protéine.

Conformément à la présente invention, de telles microcapsules peuvent être obtenues à partir de polyholosides hydrosolubles ou de dérivés hydrosolubles de polyholosides et d'au moins une protéine par mise en œuvre des trois étapes successives suivantes :

i) au moins un polyholoside hydrosoluble ou dérivés hydrosoluble de polyholoside, au moins une protéine, ainsi que la substance pharmaceutiquement active sont dissous dans une solution aqueuse alcaline ayant un pH de préférence supérieur à 10, qui est émulsifiée par dispersion au sein d'un solvant organique non miscible, par addition d'un agent émulsifiant et par agitation ;

ii) à l'émulsion ainsi obtenue, maintenue sous agitation, on ajoute un agent de réticulation en solution dans ledit solvant organique non miscible et l'on maintient l'agitation jusqu'à obtention d'une réticulation interfaciale, et

iii) on isole les microcapsules par dilution du mélange réactionnel au moyen d'un solvant ou d'un mélange de solvants approprié, suivie d'une succession d'étapes de décantation et/ou de centrifugation et d'étapes de lavage.

Dans le cadre de la présente description et des revendications, les termes polyoside, polysaccharide et polyholoside sont parfaitement synonymes.

Les polyholosides hydrosolubles ou dérivés hydrosolubles de polyholosides utilisés dans le cadre de la présente invention englobent aussi bien des polyholosides naturels hydrosolubles, par exemple la gomme arabique ou la gomme guar, que des polyholosides modifiés en vue de les rendre hydrosolubles, par exemple par traitement au moyen de solutions acides ou alcalines ou encore par greffage chimique de chaînes latérales hydrophiles. De tels traitements de modification des polyholosides en vue de les rendre hydrosolubles ou de les transformer en dérivés de polyholosides hydrosolubles sont bien connus de l'homme de l'art. A simple titre d'exemple on mentionnera l'alcali-cellulose obtenue par traitement de la cellulose à l'aide d'une lessive de soude, l'amidon soluble obtenu par dégradation de l'amidon, de même que les dextrans de bas poids moléculaire obtenus par dégradation de dextrans. on citera également l'hydroxypropylcellulose à titre d'exemple de dérivés hydrosolubles de polyholosides résultant du greffage chimique de chaînes latérales hydrophiles.

Dans le cadre de la présente invention, ces polyholosides hydrosolubles ou dérivés hydrosolubles de polyholosides peuvent être utilisés seuls ou en mélange entre eux, en association avec au moins une protéine telle que la caséine et la gélatine.

La présente invention s'applique principalement à la microencapsulation de substances pharmaceutiquement actives de caractère hydrosoluble, et en particulier aux sels alcalins d'acides carboxyliques tels que l'acide salicylique et ses dérivés.

D'autres caractéristiques et avantages de la présente invention apparaîtront à la lecture de la description détaillée faite ci-après, notamment en référence à quelques exemples particuliers de mise en œuvre du procédé de l'invention, donnés à titre de simple illustration.

Conformément à la présente invention, les microcapsules sont obtenues à partir de polyholosides hydrosolubles ou dérivés hydrosolubles de polyholosides en mélange avec au moins une protéine, et auxquels on applique le procédé de microencapsulation par émulsion-réticulation, développé par M. C. LEVY et coll. (RAMBOURG (P.) : « Sur la microencapsulation de l'invertase et de l'hémoglobine : de la polymérisation interfaciale au procédé d'émulsion-réticulation ». Thèse Doctorat, 3ᵉ cycle, Université Paris-Sud, U.E.R. Chimie Thérapeutique, 1980, LEVY (M. C.), RAMBOURG (P.) et LEVY (J.) 2ᵉ Congrès Int. Technol. Pharm. Paris 1980, III, 15-24 Microencapsulation II).

Ce procédé de microencapsulation selon l'invention se subdivise en trois étapes successives, à savoir :

i) une première étape d'émulsification du mélange de polyholosides hydrosolubles ou dérivés hydrosolubles de polyholosides et d'au moins une protéine ;

ii) une deuxième étape de réticulation interfaciale, et

iii) une troisième étape d'isolement des microcapsules.

Au cours de la première étape d'émulsification, on disperse une solution aqueuse alcaline de la substance qui sera encapsulée, du polyholoside hydrosoluble ou dérivé hydrosoluble de polyholoside ainsi que de la protéine en fins globules au sein d'un solvant organique non miscible.

L'alcalinisation de la solution aqueuse est réalisée par exemple par addition d'hydroxyde de sodium jusqu'à une concentration d'environ 0,5 M, de manière à atteindre un pH de préférence supérieur à 10.

Le solvant organique utilisé au cours de cette première étape d'émulsification est avantageusement choisi de telle sorte qu'il ne soit pas miscible à l'eau, que sa densité soit sensiblement comprise entre 0,7 et 1,1, et que l'agent de réticulation utilisé dans la deuxième phase s'y dissolve. On emploie avec profit par exemple un mélange binaire constitué par un hydrocarbure chloré à un ou deux atomes de carbone, tel que le chloroforme, le chlorure de méthylène ou le trichloréthylène, et par un hydrocarbure non chloré comportant de 5 à 7 atomes de carbone, tels que l'hexane ou le cyclohexane.

L'agent émulsifiant utilisé au cours de cette première étape est un agent tensioactif tel qu'un ester de sorbitane et d'acide gras ou tout autre tensioactif choisi par l'homme de l'art en vue de préparer une émulsion de type eau dans l'huile. Le trioléate de sorbitane (Span 85®) est utilisé avec profit. Il convient cependant de noter que, du fait de l'alcalinité de la solution à émulsifier, on évitera avantageusement les agents tensioactifs cationiques, et l'on donnera la préférence aux tensioactifs non ioniques.

Les concentrations, volumes et quantités relatifs de la solution aqueuse, de solvant organique et d'agent émulsifiant permettant d'obtenir l'émulsion susceptible d'être ultérieurement réticulée peuvent être adaptés avec une grande latitude et s'éloigner notablement des proportions indiquées dans les exemples décrits ci-après.

Une telle émulsion peut aisément être obtenue par exemple au moyen d'une hélice métallique à 5 pales (chacune de 20 mm de longueur) entraînée mécaniquement à l'intérieur d'un becher de 100 ml. Une vitesse de rotation comprise entre environ 1 000 et environ 2 000 tours par minute, poursuivie pendant au moins environ trois minutes suffit alors pour mener à bien l'émulsification.

Dans la deuxième étape de réticulation interfaciale, la paroi des microcapsules est formée par réticulation en particulier au moyen d'un réactif bifonctionnel acylant, tel qu'un halogénure de diacide carboxylique, un diisocyanate, où les deux groupements fonctionnels sont tous deux portés par un cycle aromatique, ou séparés par une chaîne aliphatique de 2 à 10 atomes de carbone, ou portés, l'un par un cycle aromatique, l'autre par une chaîne aliphatique juxtanucléaire, par exemple, le dichlorure de succinyle, le dichlorure de sébacoyle, le dichlorure de téréphtaloyle, le diisocyanate de toluène, le diisocyanate d'hexaméthylène.

L'agent de réticulation est ajouté, en solution de préférence dans le solvant organique déjà utilisé dans la première étape, à l'émulsion maintenue sous agitation.

Cette opération est réalisée à une température comprise entre le point de congélation et le point d'ébullition des liquides utilisés, le plus souvent à température ordinaire.

Une concentration de l'agent de réticulation dans le mélange solvant variant de la saturation à une concentration 0,1 M est compatible avec l'obtention de microcapsules. Des conditions favorables à la préparation des microcapsules sont généralement obtenues lorsque la solution de l'agent de réticulation est ajoutée à l'émulsion sous un volume sensiblement égal au volume de solvant ayant servi à préparer ladite émulsion.

L'agitation est poursuivie jusqu'à ce qu'un examen au microscope optique montre la formation de microcapsules individualisées, ce qui nécessite en moyenne une durée comprise entre environ 3 et environ 30 minutes.

La troisième étape d'isolement des microcapsules est réalisée en diluant le mélange réactionnel au moyen d'un solvant ou d'un mélange de solvants, puis en soumettant la suspension de microcapsules à une succession de décantations et de lavages.

La décantation peut être avantageusement remplacée par une centrifugation, par exemple 350 × g, 30 secondes.

Le lavage des microcapsules est effectué dans un solvant choisi pour ne pas entraîner les substances microencapsulées, tel que par exemple l'éther éthylique, l'alcool éthylique et l'acétone.

Les microcapsules sèches sont obtenues par évaporation de l'éther, de l'alcool ou de l'acétone.

Dans les exemples rapportés ci-après illustrant la préparation de microcapsules vides, le lavage a par exemple été effectué au moyen d'une solution de polysorbate (Tween 20®) dans l'eau distillée, puis au moyen d'eau pure. Des capsules sèches peuvent alors être obtenues après lyophilisation.

Les microcapsules à parois mixtes selon les caractéristiques de l'invention se présentent en microscopie optique sous forme de sphérules bien individualisées dont le diamètre peut varier entre 25 et 300 μm. Des sphérules de taille plus grande peuvent être obtenues lorsque les opérations d'émulsification et de réticulation sont effectuées avec une vitesse d'agitation inférieure à 1 000 tours par minute. Une membrane épaisse d'environ 1 à environ 2 μm est visible.

En microscopie électronique à balayage, elles montrent une paroi continue.

Les microcapsules préparées selon les caractéristiques de l'invention à base d'hydroxypropylcellulose résistent, sans altération, à une incubation de 10 heures en milieu gastrique artificiel (U.S.P. XIX), comme à une incubation de 10 heures en milieu intestinal artificiel (U.S.P. XIX). De telles capsules laissent lentement diffuser au travers de leur paroi le produit qui a été encapsulé. Ces propriétés s'appliquent à la préparation de médicaments sous une forme galénique à action prolongée.

Selon l'invention, des proportions variables d'une protéine telle que la caséine et la gélatine sont ajoutées aux polyholosides hydrosolubles ou dérivés hydrosolubles de polyholosides au cours de la première phase d'émulsification. On obtient ainsi des microcapsules à parois mixtes, constituées à la fois de polyholosides réticulés et de protéine réticulée. De telles microcapsules, préparées selon les caractéristiques de l'invention par exemple à partir d'hydroxypropylcellulose et de caséine, restent inaltérées après une incubation de 10 heures en milieu gastrique artificiel (U.S.P. XIX), mais leurs parois sont lysées après une incubation d'environ 6 heures en milieu intestinal artificiel (U.S.P. XIX). Les propriétés de ces microcapsules à parois mixtes selon l'invention s'appliquent plus particulièrement à la préparation de médicaments sous une forme galénique gastrorésistante entérosoluble.

Les exemples suivants illustrent l'objet de la présente invention, sans en limiter toutefois les applications.

Exemple 1

Microcapsules à parois mixtes préparées à partir d'hydroxypropylcellulose (HPC) et de caséine réticulée

Toutes les opérations sont conduites à température ordinaire.

1) Emulsification

Une solution à 3 g % (p/v) d'HPC dans la soude 0,5 N dans laquelle sont dissous 200 mg de caséine alcali-soluble est préparée par agitation magnétique à température ambiante prolongée pendant 2 heures.

Dans un becher de 100 ml, sont placés 15 ml d'un mélange chloroforme-cyclohexane (1 : 4 v/v) ci-après appelé mélange solvant-additionné de 0,30 ml trioléate de sorbitane (Span 85®). On ajoute la solution alcaline d'HPC et de caséine et on agite mécaniquement (1 000 tours par minute) au moyen d'une hélice métallique à 5 pales.

2) Réticulation

Après 3 minutes, sans interrompre l'agitation, on ajoute 20 ml d'une solution saturée de chlorure de téréphtaloyle dans le mélange solvant. L'agitation est poursuivie pendant 3 minutes.

3) Isolement des microcapsules

La réaction de polymérisation est stoppée par addition de 30 ml de mélange solvant dans le becher de réaction. Après agitation manuelle (agitateur de verre) de quelques minutes, le contenu du becher est réparti dans deux tubes à centrifuger.

Après centrifugation de 30 secondes à 350 × g, le surnageant est rejeté. Chaque culot de centrifugation est remis en suspension dans 20 ml d'une solution à 10 % (v/v) de polysorbate (Tween 20®) dans l'eau distillée puis centrifugé (30 secondes, 350 × g). Après un nouveau lavage effectué dans les mêmes conditions, les culots de centrifugation sont lavés à l'eau pure.

4) Lyophilisation

Les culots de centrifugation sont remis en suspension dans 50 ml d'eau distillée, congelés et lyophilisés.

On obtient environ 100 mg de microcapsules sèches. Les microcapsules obtenues se présentent, après réhydratation sous forme de sphères régulières de diamètre compris entre 30 et 80 μm.

La figure 1 annexée représente un cliché de microscopie optique (grossissement 300 fois) des

4

microcapsules obtenues conformément à l'exemple 1.

La figure 2 annexée représente un cliché de microscopie électronique à balayage (grossissement 450 fois) des microcapsules préparées conformément à l'exemple 1.

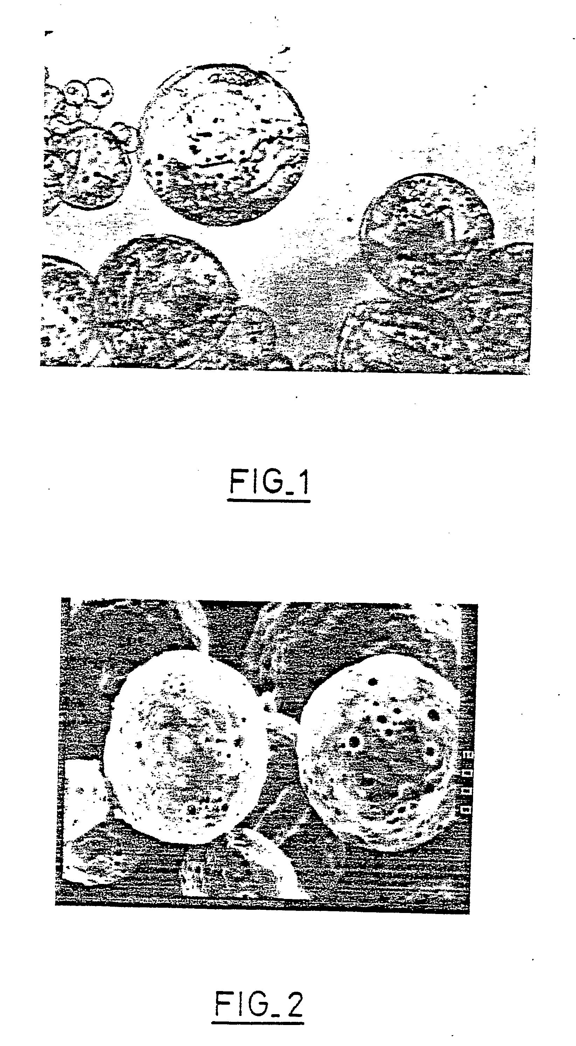

Les microcapsules se présentent en microscopie optique sous forme de sphères bien individualisées de diamètre compris entre 30 et 200 μm (voir figure 1). Une membrane est visible au microscope électronique à balayage, elles apparaissent comme des sphères non collapsées. La surface, irrégulière, présente des dépressions (voir figure 2).

### 1) Essai de gastrorésistance

L'essai poursuivi comme dans l'exemple 1 ne montre aucune altération des microcapsules après 10 heures.

### 2) Essai de solubilité entérique

Les microcapsules mises à incuber dans le liquide intestinal artificiel (U.S.P. XIX) sont progressivement détruites : la lyse commence après environ 30 minutes et s'étale progressivement sur environ 6 heures.

La lyse ne se produit pas si le milieu intestinal artificiel ne contient pas de pancréatine.

## Exemple 2

Microcapsules à parois mixtes préparées à partir d'hydroxypropylcellulose (HPC) et de caséine réticulée renfermant du bleu patenté V

Préparation des lots de microcapsules

Dans 3 ml d'une solution à 3 % (p/v) d'HPC dans la soude aqueuse 0,5 N sont dissous 200 mg de caséine alcali-soluble puis 10 mg de bleu patenté V.

Trois lots de microcapsules sont préparés (voir tableau I). Les différences portent :

sur les conditions d'émulsification :

le lot 1 est préparé avec une faible vitesse d'agitation (1 000 rpm) en présence de 2 % de Span 85® (0,30 ml pour 15 ml de mélange solvant)

les lots 2 et 3 sont préparés à 1 800 rpm en présence de 5 % de Span 85® (0,75 ml pour 15 ml de mélange solvant)

sur les temps de réticulation : 3 minutes (lots 1 et 3) ou 15 minutes (lot 2).

Essais de libération du contenu

Chaque lot de microcapsules est mis en suspension dans un litre d'eau et soumis à une agitation mécanique (40 rpm) dans un ballon maintenu à 37°. Des prélèvements sont effectués périodiquement. La suspension est filtrée sur membrane de cellulose (0,22 μm) et le liquide limpide est soumis à un dosage colorimétrique.

Résultats (consignés dans le tableau I ci-après)

Avec ce type de microcapsules à parois mixtes d'HPC et de caséine réticulées, la libération du bleu patenté s'effectue lentement puisqu'après 4 heures la quantité de colorant libéré représente au maximum 10 % de la quantité de colorant encapsulé (10 mg).

De plus, après 8 heures, 13 % au maximum de bleu patenté sont libérés (lot 3), et même après 24 heures, on n'obtient que 22 % au plus de libération (contre 48 % dans le cas des microcapsules à parois d'HPC réticulée).

La comparaison des résultats obtenus avec les trois lots indique que, contrairement à ce qui a été observé pour les microcapsules d'HPC réticulée, la taille des microcapsules intervient peu (peu de différences entre les lots 1 et 3).

Le temps de réticulation a une influence sur la libération du colorant : lorsqu'il passe de 3 minutes (lot 3) à 15 minutes (lot 2), une diminution nette de la vitesse de libération est observée. Sans doute doit-on l'attribuer à une diminution de porosité de la membrane liée à la réticulation plus importante.

Tableau I

Libération du bleu patenté incorporé dans des microcapsules à paroi mixte d'HPC et de caséine réticulées (concentrations en mg/l)

| Conditions de l'émulsification | LOT 1 | | LOT 2 | | LOT 3 | |
|---|---|---|---|---|---|---|
| | vitesse 1000 rpm | Span 85® 2% | vitesse 1800 rpm | Span 85® 5% | vitesse 1800 rpm | Span 85® 5 % |
| Temps de réticulation / Temps de prélèvement | 3 minutes | | 15 minutes | | 3 minutes | |
| 30 minutes | 0,55 mg/l | | 0,20 mg/l | | 0,40 mg/l | |
| 1 heure | 0,70 | | 0,20 | | 0,50 | |
| 2 heures | 0,80 | | 0,35 | | 0,70 | |
| 3 heures | 0,90 | | 0,50 | | 0,85 | |
| 4 heures | 1 | | 0,60 | | 0,95 | |
| 5 heures | 1,10 | | 0,65 | | 1 | |
| 6 heures | 1,15 | | 0,70 | | 1,20 | |
| 7 heures | 1,15 | | 0,80 | | 1,25 | |
| 8 heures | 1,20 | | 0,85 | | 1,30 | |
| 24 heures | 1,75 | | 1,50 | | 2,20 | |

Exemple 3

Microcapsules à parois mixtes préparées à partir d'amidon soluble et de caséine réticulée

Préparation

200 mg de caséine alcali-soluble sont ajoutés à 3 ml d'une solution d'amidon soluble à 10 g % dans la soude 0,5 N.

La solution obtenue est émulsionnée dans 15 ml de mélange chloroforme-cyclohexane (1 : 4 v/v) additionnée de 0,75 ml de Span 85® par agitation de 3 minutes à 1 800 rpm.

20 ml de solution saturée de chlorure de téréphtaloyle dans le mélange solvant sont ajoutés. Après 10 minutes d'agitation (1 800 rpm), le milieu est additionné de 30 ml de mélange solvant.

Les microcapsules, séparées par centrifugation, sont lavées :
1 fois avec une solution alcoolique de Tween 20® à 5 % (v/v)
2 fois à l'alcool
1 fois à l'eau.

Comportement dans les milieux digestifs

Milieu gastrique artificiel

Les microcapsules résistent totalement au moins 8 heures.

Milieu intestinal artificiel

Une lyse commence après environ 1 heure, et se poursuit progressivement (60 % de lyse après 8 heures).

**0 095 968**

Exemple 4

Microcapsules à parois mixtes préparées à partir de dextrans et de caséine réticulée

Le protocole décrit ci-dessus est reproduit à partir de 3 ml d'une solution de dextrans à 5 g % dans la soude 0,5 N additionnée de 200 mg de caséine alcali-soluble.

Milieu gastrique artificiel

Les microcapsules sont intactes après 8 heures d'incubation.

Milieu intestinal artificiel

La lyse commence après 1 heure, s'étale sur 6 heures (totale après 6 heures).

Exemple 5

Microcapsules à parois mixtes préparées à partir de gomme arabique et de caséine réticulée

Le protocole décrit ci-dessus est reproduit et appliqué à 3 ml d'une solution de gomme arabique à 5 g % dans la soude 0,5 N additionnée de 200 mg de caséine alcali-soluble.

Milieu gastrique artificiel

Les microcapsules sont intactes après 8 heures d'incubation.

Milieu intestinal artificiel

Lyse progressive commençant après 1 heure, totale en 6 heures.

Exemple 6

Microcapsules à parois mixtes préparées à partir de dextrans et de gélatine réticulée

150 mg de dextrans sont dissous dans 3 ml d'une solution de gélatine à 3 g % dans la soude 0,5 N. Puis les microcapsules sont préparées selon le protocole décrit ci-dessus.

Milieu gastrique artificiel

Aucune lyse n'est observée après 8 heures.

Milieu intestinal artificiel

Environ 20 % de capsules sont lysées après 8 heures.

Bien entendu, la présente invention n'est nullement limitée aux modes de mise en œuvre particuliers précédemment décrits, mais il est parfaitement possible, sans pour autant sortir du cadre de la présente invention, d'en imaginer un certain nombre de variantes. C'est ainsi que les proportions relatives du polyholoside et de la protéine de départ peuvent varier dans de larges proportions déterminées en fonction de chaque application particulière. Elles peuvent par exemple être déterminées de façon à permettre une dissolution très rapide en milieu intestinal tout en conservant une parfaite gastrorésistance. C'est en effet l'incorporation de la protéine dans la paroi des microcapsules qui est déterminante pour conférer à ces dernières l'entérosolubilité souhaitée.

**Revendications**

1. Microcapsules renfermant une substance pharmaceutiquement active, gastrorésistantes et entérosolubles, du type présentant une paroi externe obtenue par réticulation interfaciale d'un polyholoside à l'aide d'un agent de réticulation constitué par un réactif bifonctionnel acylant, caractérisées en ce que la paroi externe mixte des microcapsules est obtenue par réticulation interfaciale d'un mélange comprenant d'une part au moins un polyholoside hydrosoluble ou dérivé hydrosoluble de polyholoside et d'autre part au moins une protéine.

2. Microcapsules selon la revendication 1, caractérisées en ce que ladite protéine est de la caséine ou de la gélatine.

3. Microcapsules selon l'une des revendications 1 et 2, caractérisées en ce que leur diamètre moyen se trouve sensiblement compris entre 25 et 300 μm.

4. Microcapsules selon l'une des revendications 1 à 3, caractérisées en ce qu'elles présentent une paroi continue d'épaisseur sensiblement comprise entre 1 et 2 μm.

5. Procédé de préparation de microcapsules gastrorésistantes et entérosolubles renfermant en particulier une substance pharmaceutiquement active selon la revendication 1, caractérisé en ce que l'on prépare les microcapsules à partir d'un mélange comprenant d'une part au moins un polyholoside hydrosoluble ou dérivé hydrosoluble de polyholoside et d'autre part au moins une protéine, par mise en œuvre des trois étapes successives suivantes :

i) un mélange contenant au moins un polyholoside hydrosoluble ou dérivé hydrosoluble de polyholoside, au moins une protéine ainsi que la substance pharmaceutiquement active est dissous dans une solution aqueuse alcaline ayant un pH de préférence supérieur à 10, qui est émulsifiée par dispersion au sein d'un solvant organique non miscible, par addition d'un agent émulsifiant et par agitation ;

ii) à l'émulsion ainsi obtenue, maintenue sous agitation, on ajoute un agent de réticulation en solution dans ledit solvant organique non miscible et l'on maintient l'agitation jusqu'à l'obtention d'une réticulation interfaciale, et

iii) on isole les microcapsules par dilution du mélange réactionnel au moyen d'un solvant ou d'un mélange de solvants approprié, suivie d'une succession d'étapes de décantation et/ou de centrifugation et d'étapes de lavage.

6. Procédé selon la revendication 5, caractérisé en ce que les polyholosides hydrosolubles ou dérivés hydrosolubles de polyholosides sont en particulier choisis parmi les polyholosides naturels hydrosolubles, les polyholosides rendus hydrosolubles par traitement à l'aide de solutions acides ou alcalines ou encore par greffage chimique de chaînes latérales hydrophiles, ainsi que leurs mélanges.

7. Procédé selon la revendication 6, caractérisé en ce que les polyholosides hydrosolubles ou dérivés hydrosolubles de polyholosides sont choisis parmi l'alcalicellulose, l'amidon soluble, les dextrans de bas poids moléculaire, l'hydroxypropylcellulose, la gomme arabique, la gomme guar ainsi que leurs mélanges.

8. Procédé selon l'une des revendications 5 à 7, caractérisé en ce que la protéine est constituée par de la caséine.

9. Procédé selon l'une des revendications 5 à 7, caractérisé en ce que la protéine est constituée par de la gélatine.

10. Procédé selon l'une des revendications 5 à 9, caractérisé en ce que le solvant organique utilisé au cours de l'étape d'émulsification est un solvant non miscible à l'eau, de densité sensiblement comprise entre 0,7 et 1,1, et permettant la dissolution de l'agent de réticulation utilisé au cours de la seconde étape de réticulation interfaciale.

11. Procédé selon l'une des revendications 5 à 10, caractérisé en ce que l'agent émulsifiant utilisé au cours de l'étape d'émulsification est un agent tensioactif, de préférence de type non ionique, permettant la préparation d'une émulsion du type eau dans huile.

12. Procédé selon l'une des revendications 5 à 11, caractérisé en ce que ledit solvant organique non miscible est constitué par un mélange binaire d'un hydrocarbure chloré en $C_1$ ou $C_2$ et d'un hydrocarbure non chloré en $C_5$ à $C_7$.

13. Procédé selon l'une des revendications 5 à 12, caractérisé en ce que l'agent de réticulation est un réactif bifonctionnel acylant, tel qu'un halogénure de diacide carboxylique, un diisocyanate, où les deux groupements fonctionnels sont tous deux portés par un cycle aromatique ou séparés par une chaîne aliphatique contenant de 2 à 10 atomes de carbone, ou portés, l'un par un cycle aromatique et l'autre par une chaîne aliphatique juxtanucléaire.

14. Procédé selon l'une des revendications 5 à 13, caractérisé en ce que l'agent de réticulation est choisi parmi le dichlorure de succinyle, le dichlorure de sébacoyle, le dichlorure de téréphtaloyle, le diisocyanate de toluène et le diisocyanate d'hexaméthylène.

15. Procédé selon l'une des revendications 5 à 14, caractérisé en ce que l'agent de réticulation est ajouté au mélange réactionnel en solution dans le même solvant organique que celui utilisé au cours de la première étape d'émulsification.

16. Procédé selon l'une des revendications 13 à 15, caractérisé en ce que la concentration de l'agent de réticulation dans ledit solvant organique varie de la saturation à une concentration 0,1 M.

17. Procédé selon la revendication 16, caractérisé en ce que la solution d'agent de réticulation est ajoutée à l'émulsion à raison d'un volume sensiblement égal au volume de solvant utilisé pour la préparation de ladite émulsion.

18. Procédé selon l'une des revendications 5 à 17, caractérisé en ce que les microcapsules sont récupérées à l'état sec, soit par évaporation du ou des solvants de lavage, soit par lyophilisation de la suspension des microcapsules dans l'eau.

## Claims

1. Microcapsules containing a pharmaceutically active substance, which microcapsules are resistant to gastric juices and enterosoluble, of the type having an outer wall obtained by the interfacial reticulation of a polyholoside with the aid of a reticulating agent constituted by a bifunctional acylating reagent, characterised in that the composite external wall of the microcapsules is obtained by interfacial reticulation of a mixture comprising, on the one hand, at least one water-soluble polyholoside or water-soluble polyholoside derivative and, on the other hand, at least one protein.

2. Microcapsules according to claim 1, characterised in that said protein is casein or gelatine.

3. Microcapsules according to either of claims 1 and 2, characterised in that their mean diameter is substantially comprised between 25 and 30 $\mu$m.

4. Microcapsules according to one of claims 1 to 3, characterised in that they have a continuous wall of a thickness substantially comprised between 1 and 2 $\mu$m.

5. Method of preparation of microcapsules resistant to gastric juices and enterosoluble, comprising in particular a pharmaceutically active substance according to claim 1, characterised in that the microcapsules are prepared from a mixture comprising, on the one hand, at least one water-soluble polyholoside or water-soluble polyholoside derivative and, on the other hand, at least one protein, by means of the implementation of the three successive stages following :

i) a mixture containing at least one water-soluble polyholoside or water-soluble polyholoside derivative, at least one protein as well as the pharmaceutically active substance is dissolved in an aqueous alkaline solution having a pH preferably in excess of 10, which is emulsified by dispersion within a non-miscible organic solvent, by the addition of an emulsifying agent and by stirring ;

ii) to the emulsion thus obtained, still undergoing stirring, the addition is made of a reticulation agent dissolved in said non-miscible organic solvent, and stirring is continued until interfacial reticulation is obtained, and

iii) the microcapsules are isolated by dilution of the reaction mixture by means of a solvent or a suitable mixture of solvents, followed by a succession of decanting and/or centrifuging steps and of washing steps.

6. Method according to claim 5, characterised in that the water-soluble polyholosides or water-soluble polyholoside derivatives are selected in particular from among the naturally water-soluble polyholosides, the polyholosides made water-soluble by processing with acid or alkaline solutions, or also by chemical grafting of hydrophilous side chains, as well as their mixtures.

7. Method according to claim 6, characterised in that the water-soluble polyholosides or water-soluble polyholoside derivatives are chosen from alkali-cellulose, soluble starch, low-molecular weight dextrans, hydroxypropylcellulose, gum arabic, guar gum as well as the mixtures thereof.

8. Method according to one of claims 5 to 7, characterised in that the protein consists of casein.

9. Method according to one of claims 5 to 7, characterised in that the protein consists of gelatine.

10. Method according to one of claims 5 to 9, characterised in that the organic solvent used during the emulsifying step is a non-water-miscible solvent, of a density substantially comprised between 0.7 and 1.1, and permitting the dissolution of the reticulation agent used in the course of the second step of interfacial reticulation.

11. Method according to one of claims 5 to 10, characterised in that the emulsifying agent used during the emulsification step is a surface-active agent, preferably of non-ionic type, permitting the preparation of a water-in-oil type emulsion.

12. Method according to one of claims 5 to 11, characterised in that said non-miscible organic solvent consists of a binary mixture of $C_1$ or $C_2$ chlorinated hydrocarbon and of a $C_5$ to $C_7$ non-chlorinated hydrocarbon.

13. Method according to one of claims 5 to 12, characterised in that the reticulation agent is an acylating bi-functional reagent, such as a carboxylic diacid halide, a diisocyanate, where both functional groups are carried by an aromatic cycle or separated by an aliphatic chain containing from 2 to 10 carbon atoms, or carried, one by an aromatic cycle and the other by a juxta-nuclear aliphatic chain.

14. Method according to one of claims 5 to 13, characterised in that the reticulation agent is selected from succinyl dichloride, sebacyl dichloride, terephthalyl dichloride, toluene diisocyanate and hexamethylene diisocyanate.

15. Method according to one of claims 5 to 14, characterised in that the reticulation agent is added to the reaction mixture in solution in the same organic solvent as that used during the first emulsifying step.

16. Method according to one of claims 13 to 15, characterised in that the concentration of the reticulation agent in said organic solvent varies from saturation to a concentration of 0.1 M.

17. Method according to claim 16, characterised in that the solution of reticulation agent is added to the emulsion at the rate of a volume substantially equal to the volume of solvent used for the preparation of said emulsion.

18. Method according to one of claims 5 to 17, characterised in that the microcapsules are recovered in the dry state, either by evaporation of the washing solvent(s), or by lyophilisation of the suspension of microcapsules in water.

**Patentansprüche**

1. Gastroresistente und enterolösliche Mikrokapseln, die eine pharmazeutisch wirksame Substanz

**0 095 968**

enthalten, die in der Form vorliegen, daß eine äußere Wand, die durch Inter-Oberflächenvernetzung eines Polyholosids mit Hilfe eines Vernetzungsagens, das aus einem bifunktionellen Acylierungsagens besteht, erhalten wird, und dadurch gekennzeichnet sind, daß die äußere gemischte Wand der Mikrokapseln durch Inter-Oberflächenvernetzung einer Mischung erhalten wird, die zu einem Teil zumindestens ein wasserlösliches Polyholosid oder ein wasserlösliches Polyholosidderivat und zum anderen Teil mindestens ein Protein enthält.

2. Mikrokapseln nach Anspruch 1, dadurch gekennzeichnet, daß das besagte Protein Kasein oder Gelatin ist.

3. Mikrokapseln nach einem der Ansprüche 1 und 2, dadurch gekennzeichnet, daß ihr Durchmesser im wesentlichen zwischen 25 und 300 μm beträgt.

4. Mikrokapseln nach einem der Ansprüche 1 bis 3, dadurch gekennzeichnet, daß sie eine Wand aufweisen, die eine Stärke von im wesentlichen zwischen 1 und 2 μm aufweisen.

5. Verfahren zur Herstellung von gastroresistenten und enterolöslichen Mikrokapseln, die im besonderen eine pharmazeutisch wirksame Substanz nach Anspruch 1 enthalten, dadurch gekennzeichnet, daß man die Mikrokapseln ausgehend von einer Mischung herstellt, die zu einem Teil mindestens ein wasserlösliches Polyholosidderivat und zum anderen Teil mindestens ein Protein enthält, durch sukzessive Durchführung der folgenden drei Schritte :

i) eine Mischung, die mindestens ein wasserlösliches Polyholosid oder ein wasserlösliches Polyholosidderivat, mindestens ein Protein, sowie die pharmazeutisch wirksame Substanz enthält, wird in einer wäßrigen alkalischen Lösung mit einem pH, der höher als 10 ist, gelöst, welche durch Dispersion in einem nicht mischbaren organischen Lösungsmittel emulgiert wird, durch die Zugabe eines Emulgierungsmittels und durch Rühren ;

ii) der so erhaltenen Emulsion, welche unter Rühren gehalten wird, wird ein Vernetzungsagens in dem besagten nicht mischbaren organischen Lösungsmittel hinzugefügt, und weiter gerührt wird, bis eine Inter-Oberflächenvernetzung erhalten wird, und

iii) die Mikrokapseln werden durch Verdünnung des Reaktionsgemisches mit einem Lösungsmittel oder einer geeigneten Mischung von Lösungsmitteln isoliert, gefolgt von einer Reihe von Dekantierungs- und/oder Zentrifugationsschritten und von Waschschritten.

6. Verfahren nach Anspruch 5, dadurch gekennzeichnet, daß die wasserlöslichen Polyholoside oder die wasserlöslichen Polyholosidderivate im besonderen ausgewählt werden aus den natürlichen wasserlöslichen Polyholosiden, den Polyholosiden, die durch Behandlung mit sauren oder alkalischen Lösungen, oder auch durch chemisches Pfropfen der hydrophilen Seitenketten wasserlöslich gemacht werden, sowie deren Mischungen.

7. Verfahren nach Anspruch 6, dadurch gekennzeichnet, daß die wasserlöslichen Polyholoside oder die wasserlöslichen Polyholosidderivate ausgewählt werden von Alkali-Zellulose, löslicher Stärke, den Dextranen mit niedrigem Molekulargewicht, Hydroxypropylzellulose, Gummiarabikum, Guargummi, sowie deren Mischungen.

8. Verfahren nach einem der Ansprüche 5 bis 7, dadurch gekennzeichnet, daß das Protein aus Kasein besteht.

9. Verfahren nach einem der Ansprüche 5 bis 7, dadurch gekennzeichnet, daß das Protein aus Gelatine besteht.

10. Verfahren nach einem der Ansprüche 5 bis 9, dadurch gekennzeichnet, daß das im Zuge des Emulgierungsschrittes verwendete organische Lösungsmittel ein mit Wasser nicht mischbares Lösungsmittel ist, mit einer Dichte die im wesentlichen zwischen 0,7 und 1,1 liegt und das die Lösung des im Zuge des zweiten Schrittes der Inter-Oberflächenvernetzung verwendeten Vernetzungsagens erlaubt.

11. Verfahren nach einem der Ansprüche 5 bis 10, dadurch gekennzeichnet, daß das Emulgierungsagens, das im Zuge des Emulgierungsschrittes verwendet wird, ein tensioaktives Agens ist, vorzugsweise von nicht ionischem Typ, das die Herstellung einer Emulsion vom Typ Wasser in Öl erlaubt.

12. Verfahren nach einem der Ansprüche 5 bis 11, dadurch gekennzeichnet, daß das besagte nicht mischbare organische Lösungsmittel aus einer binären Mischung eines chlorierten Kohlenwasserstoffs mit $C_1$ oder $C_2$ und eines nicht chlorierten Kohlenwasserstoffs mit $C_5$ bis $C_7$ besteht.

13. Verfahren nach einem der Ansprüche 5 bis 12, dadurch gekennzeichnet, daß das Vernetzungsagens ein bifunktionelles Acylierungsreagens ist, wie ein Halogenid einer zweibasigen Carbonsäure, ein Diisocyanat, bei welchem die beiden funktionellen Gruppen beide von einem aromatischen Ring getragen werden oder durch eine aliphatische Kette mit 2 bis 10 Kohlenstoffatomen getrennt sind, oder eine von einem aromatischen Ring und die andere von einer dem Kern gegenüberliegenden aliphatischen Kette getragen sind.

14. Verfahren nach einem der Ansprüche 5 bis 13, dadurch gekennzeichnet, daß das Vernetzungsagens ausgewählt wird von Succinyldichlorid, Sebacoyldichlorid, Terephthaloyldichlorid, Toluoldiisocyanat und Hexamethylendiisocyanat.

15. Verfahren nach einem der Ansprüche 5 bis 14, dadurch gekennzeichnet, daß das Vernetzungsagens einem Reaktionsgemisch in Lösung in dem gleichen organischen Lösungsmittel, welches im Zuge des ersten Schrittes der Emulgierung verwendet wird, hinzugefügt wird.

16. Verfahren nach einem der Ansprüche 13 bis 15, dadurch gekennzeichnet, daß die Konzentration des Vernetzungsagens in dem besagten organischen Lösungsmittel von der Sättigung bis zu einer

10

**0 095 968**

Konzentration von 0,1 M variiert.

17. Verfahren nach Anspruch 16, dadurch gekennzeichnet, daß ein Volumen der Lösung des Vernetzungsagens der Emulsion hinzugefügt wird, das im wesentlichen gleich ist dem Volumen des für die Herstellung der besagten Emulsion verwendeten Lösungsmittel.

18. Verfahren nach einem der Ansprüche 5 bis 17, dadurch gekennzeichnet, daß die Mikrokapseln in trockenem Zustand gewonnen werden, sei es durch Verdampfen des oder der Lösungsmittel des Waschvorganges, oder durch Lyophilisierung der Suspension der Mikrokapseln in Wasser.

FIG_1

FIG_2